# DEMANDE DE BREVET EUROPEEN

(11) **EP 2 292 262 A2**
(43) Date de publication de la demande: **09.03.2011**
(21) Numéro de dépôt: 10182238.5
(22) Date de dépôt: 07.05.2003
(51) Int. Cl.: A61K 39/205, C12N 15/47, C12N 7/04, C12N 15/86

(54) **Utilisation de novirhabdovirus modifiés pour l'obtention de vaccins**

(30) Priorité: 07.05.2002 FR 0205702
(62) Demande divisionnaire de: 03752789.2
(71) Demandeur: Institut National De La Recherche Agronomique, 75007 Paris (FR)
(72) Inventeur: Bremont, Michel, 94600 Choisy-le-Roi (FR); Biacchesi, Stéphane, 94350 Villiers-sur-Marne (FR); Leberre, Monique, 78180 Montigny-le-Bretonneux (FR); Thoulouze, Maria-Isabel, 75013 Paris (FR)
(74) Mandataire: Vialle-Presles, Marie José

(57) **Abrégé**

L'invention est relative à l'utilisation de novirhabdovirus modifiés chez lesquels le gène de la protéine NV est inactivé pour l'obtention de vaccins utilisables notamment chez les poissons.

## Description

La présente invention est relative à l'utilisation de novirhabdovirus pour la production de vaccins.

Les novirhabdovirus sont des virus à ARN négatif de la famille des *Rhabdoviridae.*

Le genre *Novirhabdovirus* comprend différentes espèces pathogènes des animaux aquatiques, parmi lesquelles on citera notamment deux espèces pathogènes des poissons, et principalement des salmonidés : le virus de la nécrose hématopoïétique infectieuse (VNHI) et le virus de la septicémie hémorragique virale (VSHV).

La structure du génome des novirhabdovirus est proche de celle des rhabdovirus de mammifères, mais se différencie de celle-ci par la présence d'un gène supplémentaire, codant pour une protéine non-structurale, dénommée protéine NV (pour « non-virion »), dont la fonction demeure inconnue à l'heure actuelle.

Le génome des novirhabdovirus comprend six gènes, dont l'organisation peut être schématisée comme suit :
3'-N-P-M-G-NV-L-5'

N représente le gène codant pour la nucléoprotéine associée à l'ARN viral, P représente le gène codant pour la phosphoprotéine associée à la polymérase virale, M représente le gène codant pour la protéine de matrice, G représente le gène codant pour la glycoprotéine d'enveloppe G, NV représente le gène codant pour la protéine NV, et L représente le gène codant pour l'ARN polymérase virale ARN dépendante.

Le VNHI et le VSHV provoquent des dégâts importants dans les piscicultures, en particulier chez les alevins. Des vaccins capables de conférer une immunité protectrice contre ces virus ont été proposés. Ils sont basés soit sur l'utilisation de virus tués ou inactivés, soit sur l'utilisation de la glycoprotéine G, qui peut induire la synthèse d'anticorps neutralisants capables de conférer une immunité protectrice. Il a ainsi été proposé d'utiliser des vaccins à base de glycoprotéine G recombinante, ou des vaccins d'ADN nu porteur du gène codant pour la glycoprotéine G. Toutefois, ces vaccins doivent être administrés par injection, et il est très difficile en pratique d'utiliser cette méthode d'administration sur des milliers de jeunes alevins.

Des travaux précédents de l'équipe des Inventeurs (BIACCHESI et al., J. Virol., 74, 11247-11253, 2000) ont montré que lorsque le gène NV du virus NHI était inactivé et remplacé par un gène rapporteur tel que le gène codant pour la GFP (green fluorescent protein), les virus recombinants ainsi produits conservaient leur pouvoir infectieux et pouvaient se multiplier normalement en culture de cellules.

Les Inventeurs ont maintenant découvert que, de manière surprenante, ces virus NHI recombinants dépourvus du gène de la protéine NV perdent totalement leur pouvoir pathogène. Ils ont en outre constaté que si l'on insère à la place du gène NV d'origine, le gène NV du virus SHV, la pathogénicité du virus est rétablie ; en revanche, si l'on insère à la place du gène NV d'origine, le gène de la glycoprotéine G du virus SHV, on obtient des virus infectieux non-pathogènes capables d'induire une immunité protectrice à la fois contre le virus NHI et le virus SHV. En outre, ils ont constaté que bien que les virus NHI recombinants dépourvus du gène de la protéine NV conservent leur capacité de multiplication en culture de cellules, ils ne se multipliaient pas chez le poisson (ou étaient éliminés très rapidement).

Cette découverte permet de proposer l'utilisation de novirhabdovirus chez lesquels le gène de la protéine NV est inactivé pour exprimer *in vivo* un ou plusieurs gènes exogènes d'intérêt, chez un poisson.

La présente invention a plus particulièrement pour objet l'utilisation de novirhabdovirus chez lesquels le gène de la protéine NV est inactivé pour l'obtention de médicaments.

On entend par « novirhabdovirus chez lequel le gène NV est inactivé » tout novirhabdovirus portant une mutation qui entraîne l'absence de production de la protéine NV, ou la production d'une protéine NV non-fonctionnelle, sans abolir la production ou la fonction des autres protéines virales.

Le gène de la protéine NV peut par exemple être inactivé par la délétion d'au moins une partie dudit gène ou, avantageusement de la totalité de celui-ci, ou éventuellement par mutation non-sens, décalage du cadre de lecture, etc. On préférera généralement effectuer l'inactivation par délétion de la totalité ou d'une portion importante du gène, ce qui permet d'éviter tout risque de réversion vers le virus sauvage.

En particulier, la présente invention a pour objet tout novirhabdovirus recombinant dont le gène de la protéine NV est inactivé, et qui contient dans son génome au moins un gène hétérologue codant pour une protéine d'intérêt thérapeutique.

Selon un mode de réalisation préféré de la présente invention, ladite protéine d'intérêt thérapeutique est choisie parmi des antigènes vaccinaux d'organismes, et notamment de virus, pathogènes pour des poissons, et notamment parmi les glycoprotéines de capside ou d'enveloppe de virus pathogènes des poissons. A titre d'exemples non-limitatifs on citera la glycoprotéine E2 et la glycoprotéine E1 du virus de la maladie du sommeil des salmonidés (VILLOING et al., J. Virol. 74, 173-183, 2000, et Dis. Aquat. Org., 40, 19-27, 2000) ou la glycoprotéine G d'un rhabdovirus, en particulier la glycoprotéine G d'un novirhabdovirus d'une espèce autre que celle dont est issue le novirhabdovirus recombinant conforme à l'invention.

Selon un mode de réalisation préféré de la présente invention, ledit gène hétérologue est inséré à l'emplacement du gène inactivé de la protéine NV.

Avantageusement, un novirhabdovirus recombinant conforme à l'invention est choisi parmi :
- un virus NHI dont le gène de la protéine NV est inactivé, et remplacé par le gène de la glycoprotéine G d'un virus SHV ;
- un virus SHV dont le gène de la protéine NV est inactivé, et remplacé par le gène de la glycoprotéine G d'un virus NHI.

Des novirhabdovirus recombinants conformes à l'invention peuvent être obtenus par des méthodes connues en elles-mêmes, notamment par la méthode décrite dans le Brevet US 6033886 pour la préparation des rhabdovirus, qui consiste à co-transfecter une cellule-hôte exprimant une ARN polymérase avec l'ADN complémentaire (ADNc) du génome viral, et des molécules d'ADN codant pour les protéines virales N, P, et L.

L'ADNc du génome d'un novirhabdovirus recombinant conforme à l'invention est obtenu à partir de l'ADNc du virus sauvage correspondant, par introduction d'une ou plusieurs mutations inactivant le gène NV, et insertion du gène hétérologue d'intérêt. Ces modifications peuvent être effectuées par les techniques classiques du génie génétique.

La présente invention englobe également l'ADNc du génome d'un novirhabdovirus recombinant conforme à l'invention, ainsi que tout vecteur recombinant comprenant ledit ADNc.

La présente invention a également pour objet les médicaments comprenant un novirhabdovirus chez lequel le gène NV est inactivé, et notamment les vaccins comprenant un novirhabdovirus recombinant conforme à l'invention.

Les vaccins conformes à l'invention peuvent être utilisés chez les poissons, et en particulier les salmonidés, tels que les truites et les saumons d'élevage.

L'utilisation de novirhabdovirus recombinants conformes à l'invention, qui expriment simultanément la glycoprotéine G du novirhabdovirus d'origine, et au moins un antigène vaccinal issu d'un autre organisme pathogène, permet d'obtenir des vaccins multivalents, capable de conférer une protection vis-à-vis d'au moins deux pathogènes. Par exemple, un novirhabdovirus recombinant conforme à l'invention qui exprime, outre la glycoprotéine G du novirhabdovirus dont il est issu, la glycoprotéine G d'un novirhabdovirus d'une espèce différente permet de conférer une protection vis-à-vis des deux espèces de novirhabdovirus concernées.

En outre, contrairement aux vaccins proposés dans l'art antérieur, qui ne sont administrables que par injection, les vaccins conformes à l'invention présentent l'avantage de pouvoir également être administrés par balnéation, c'est à dire par simple addition du vaccin à l'eau du bassin d'élevage où se trouvent les animaux à vacciner. Cette méthode d'administration est donc beaucoup plus simple à mettre en oeuvre que la méthode par injection. En outre, elle permet de résoudre les problèmes posés par la vaccination des jeunes alevins, qui sont les plus sensibles aux maladies induites par les novirhabdovirus, mais également au stress lié à la vaccination par injection.

D'autre part, l'absence de multiplication chez le poisson des novirhabdovirus recombinants conformes à l'invention permet leur utilisation sans risque de dissémination dans l'environnement et de contamination des espèces sauvages.

La présente Invention sera mieux comprise à l'aide du complément de description qui va suivre, qui se réfère à des exemples non-limitatifs de construction et d'utilisation de novirhabdovirus recombinants conformes à l'invention.

### EXEMPLE 1 : CONSTRUCTION DE NOVIRHABDOVIRUS RECOMBINANTS PAR REMPLACEMENT DU GENE NV PAR UN GENE HETEROLOGUE Construction des ADNc recombinants :

Les constructions ont été effectuées à partir du plasmide pINHV, décrit par BIACCHESI et al. (J. Virol. 2000 74(23):11247-53). Ce plasmide contient l'ADNc complet du génome du virus NHI, cloné en aval du promoteur de l'ARN polymérase du phage T7 et en amont d'une séquence ribozyme du virus de l'hépatite δ et du terminateur de transcription de l'ARN polymérase du phage T7, dans le vecteur pBlueScript SK (STRATAGENE).

Un virus NHI recombinant dans lequel le gène NV a été excisé et remplacé par le gène rapporteur GFP (Green Fluorescent Protein) a été construit comme décrit par BIACCHESI et al. (publication précitée).

### Schématiquement :

Un site de restriction SpeI et un site de restriction *Sma*I ont été introduits par mutagenèse dirigée (QUICKCHANGE kit, STRATAGENE) dans un fragment EagI-PstI de l'ADNc du génome du virus NHI de part et d'autre du cadre ouvert de lecture de NV. Ce cadre ouvert de lecture a été excisé par digestion *Spe*I*-Sma*I à partir de ce fragment, et remplacé par le gène rapporteur GFP. Le fragment *Eag*I-*Pst*I modifié a été introduit dans le plasmide pINHV, à la place de la région correspondante de celui-ci.

Le plasmide obtenu est dénommé pINHV-ΔNV-GFP

Des virus NHI recombinants dans lesquels le gène NV a été remplacé par le gène NV ou le gène G de SHV ont été construits comme suit :
Les gènes NV et G ont été obtenus à partir de la souche 07-71 de SHV (SCHUETZE H. et al. Virus Genes., 19(1), 59-65, 1999; GenBank NC 000855), en utilisant les amorces suivantes:
   Gène NV :
      Amorce 3' :
      SmaI
      CCCGGGTCAGGAGGTGAGCCCAGAGCC (SEQ ID NO : 1)
      Amorce 5' :
      SpeI
      ACTAGTATGGCGACCCAACCCGGGCTCAGC (SEQ ID NO : 2)
   Gène G :
      Amorce 3' :
      SmaI
      CCCGGGTCAGACCGTCTGACTTCTGGAGAACT (SEQ ID NO : 3)
      Amorce 5' :
      SpeI
      ACTAGTATGGAATGGAACACTTTTTTCTTGG (SEQ ID NO : 4)

Des cellules épithéliales de carpe (EPC : epithelioma papulosum cyprinid) sont infectées par le virus SHV à une multiplicité d'infection de 1. Vingt quatre heures post-infection, les cellules sont lysées et les ARNs totaux sont extraits (kit RNAgents Total RNA Isolation System, PROMEGA). Approximativement 1 µg d'ARNs sont dénaturés 10mn à température ambiante en présence de 40 mM d'hydroxy-méthyl-mercure. L'ARN dénaturé est alors placé dans un mélange réactionnel contenant 80 mM de β-mercaptoéthanol, 10 mM de dithiothréitol, 1 mM de dNTP, 40 U RNasin (GIBCO-BRL), 25 pmol de l'amorce 3'G VHSV ou de l'amorce 3'NV VHSV, et 200 U de SUPERSCRIPT II, dans du tampon IX (GIBCO-BRL). Le mélange est maintenu pendant 1 heure à 42°C.

Les ADNc obtenus sont amplifiés par PCR avec l'amorce 5'G VHSV ou l'amorce 5'NV VHSV, en présence de TAQ polymérase (GIBCO BRL). Les produits PCR ainsi obtenus sont purifiés et sous-clonés dans un plasmide TA-cloning (pGEM-T Vector System, PROMEGA).

Après séquençage les fragments d'ADN G ou NV VHSV sont excisés par digestion *Spe*I et *Sma*I*.*

L'un ou l'autre de ces fragments est inséré à la place du gène NV dans le fragment *Eag*I*-Pst*I de l'ADNc du génome du virus NHI, et le fragment *Eag*I*-Pst*I ainsi modifié introduit dans le plasmide pINHV comme indiqué ci-dessus.

Les plasmides obtenus sont respectivement dénommés pINHV-ΔNV-NVSHV et pINHV-ΔNV-GSHV.

### Production des virus recombinants :

Trois plasmides d'expression comportant respectivement les gènes codant pour la nucléoprotéine N, la phosphoprotéine P, et l'ARN polymérase ARN dépendante L de NHI ont été construits, comme décrit par BIACCHESI et al. (publication précitée). Ces constructions sont respectivement appelées pT7-N, pT7-P et pT7-L.

Le plasmide pINHV, le plasmide pINHV-ΔNV-NVSHV ou le plasmide pINHV-ΔNV-GSHV, et les 3 plasmides, pT7-N, pT7-P, pT7-L, à des doses respectives de 1 µg, 0,5 µg, 0,2 µg, 0,2 µg) sont introduits par transfection en présence de Lipofectamine (GIBCO-BRL) dans des cellules EPC préalablement infectées par un virus de la vaccine recombinant exprimant l'ARN polymérase du phage T7 (vTF7-3, FUERST et al. Proc. Natl. Acad. Sci. USA, 92, 4477-4481, 1986).

Après transfection, les cellules sont incubées pendant 5 heures à 37°C puis lavées avec du milieu de culture MEM (sans sérum) et incubées pendant 7 jours à 14 °C en milieu de culture MEM contenant 2% de sérum foetal de veau. Les cellules et le surnageant sont congelés/décongelés, et clarifiés par centrifugation pendant 10 minutes à 10000 tours/min. Le surnageant est utilisé à la dilution 1/10 pour infecter un tapis de cellules EPC. Les virus sont produits dans le surnageant 3-4 jours post-infection.

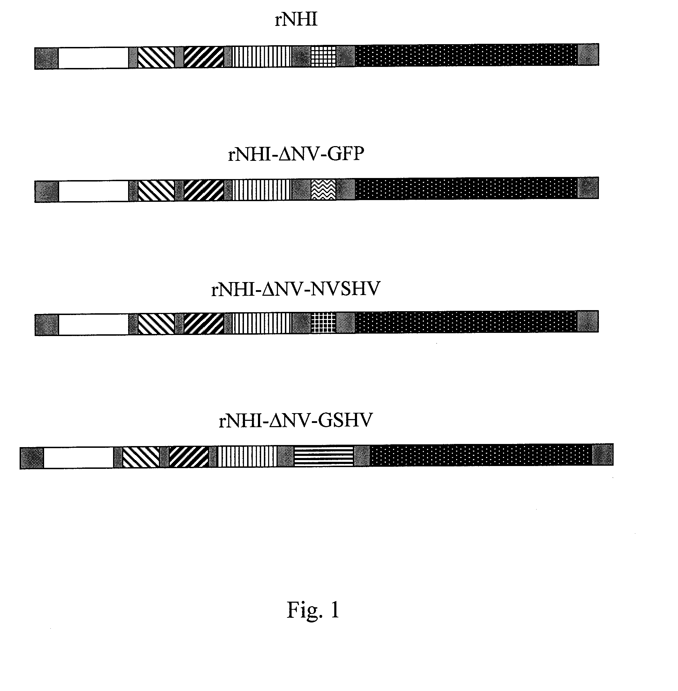

La structure des virus obtenus, possédant la totalité du génome du virus sauvage (rNHI), ou résultant de la délétion du gène NV et de son remplacement par le gène codant pour la GFP (rNHI-ΔNV-GFP), le gène NV de VSHV (rNHI-ΔNV-NVSHV), ou le gène G de VSHV (rNHI-ΔNV-GSHV), est schématisée sur la Figure 1.

Légende de la Figure 1 :
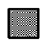 Régions intergéniques
□ Gène N
■ Gène P
□ Gène M
■ Gène G NHI
■ Gène G SHV
■ Gène NV NHI
□ Gène NV SHV
■ Gène GFP

Des stocks viraux de chacun des virus produits ont été constitués par passages successifs en culture cellulaire (EPC) du surnageant prélevé 7 jours après transfection (surnageant PO). Les cellules sont infectées à une multiplicité d'infection (m.i) de 1. Après 3 passages, les surnageants sont prélevés à différents temps post-infection, et titrés par dilution limite pour établir une courbe de croissance.

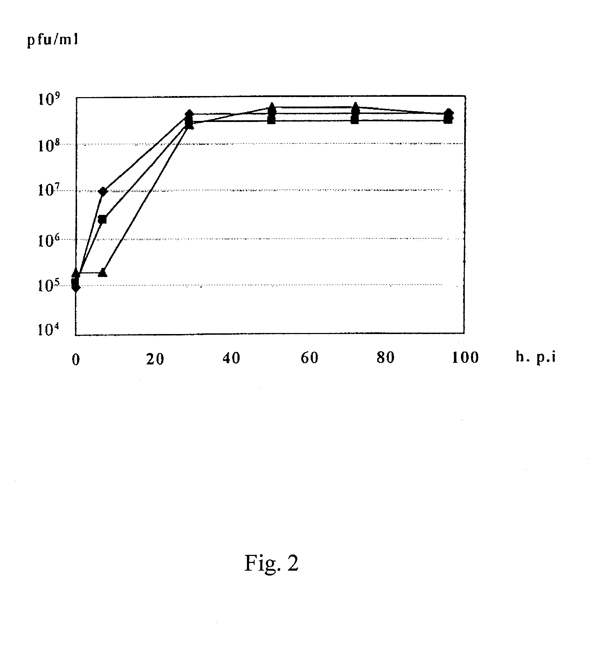

Les courbes de croissance pour les virus SHV et NHI sauvages, et pour le virus recombinant VNHI-ΔNV-GVSHV sont représentées sur la Figure 2. Ces courbes montrent que le virus recombinant VNHI-ΔNV-GVSHV se multiplie en culture cellulaire aussi bien que les virus sauvages NHI ou SHV. Légende de la Figure 2:
◆ : virus SHV sauvage ;
■ : virus NHI sauvage ;
▲ : virus recombinant VNHI-ΔNV-GVSHV.

### EXEMPLE 2 : PATHOGENICITE ET PROPRIETES VACCINALES DES NOVIRHABDOVIRUS RECOMBINANTS

### Pathogénicité

La pathogénicité des différents novirhabdovirus obtenus comme décrit à l'Exemple 1 ci-dessus a été évaluée par infections expérimentales chez la truite arc en ciel *(Oncorhyncus mykiss).*

### Infection par injection :

Des lots de 25 truitelles/bac (Poids moyen 2 gr) ont été infectés par injection des différents virus testés à raison de 10⁶ pfu/poisson. Les virus utilisés sont les suivants :
Virus NHI sauvage, souche française 32/87 (wt 32/87) ;
Virus NHI de type sauvage produit comme décrit à l'Exemple 1 (rNHI) ;
Virus NHI-ΔNV-GFP (rNHI-ΔNV-GFP) ;
Virus NHI-ΔNV-NVSHV (rNHI-ΔNV-NVSHV) ;
Virus NHI- ΔNV-GSHV (rNHI-ΔNV-GSHV) ;

La mortalité est suivie sur une période de 4 semaines.

Les résultats observés sur 2 expérimentations indépendantes sont résumés dans le Tableau I ci-dessous.

**Tableau I**

| **VIRUS** | **Mortalité*** |
|---|---|
| **wt 32/87** | **++** |
| **rNHI** | **++** |
| **RNHI-ΔNV-GSHV** | - |
| **RNHI-ΔNV-GFP** | - |
| **RNHI-ΔNV-NVSHV** | **++** |

| | |
|---|---|
| *Mortalités cumulées entre 7 et 15 jours post infection (++) 90-100% ; (-) <10%. | |

### Infection par balnéation :

Des alevins de truite ont été infectés par balnéation, selon le protocole suivant :
Les alevins ont placés dans des bassins d'élevage dans un volume d'eau réduit (3 litres d'eau pour 100 à 150 alevins). Le virus à tester est ajouté à l'eau du bassin à raison de 10⁴ à 5 x 10⁴ UFP/ml (UFP=unité formant plage). Après 3 heures d'incubation, les bassins sont remplis et la circulation d'eau est rétablie.

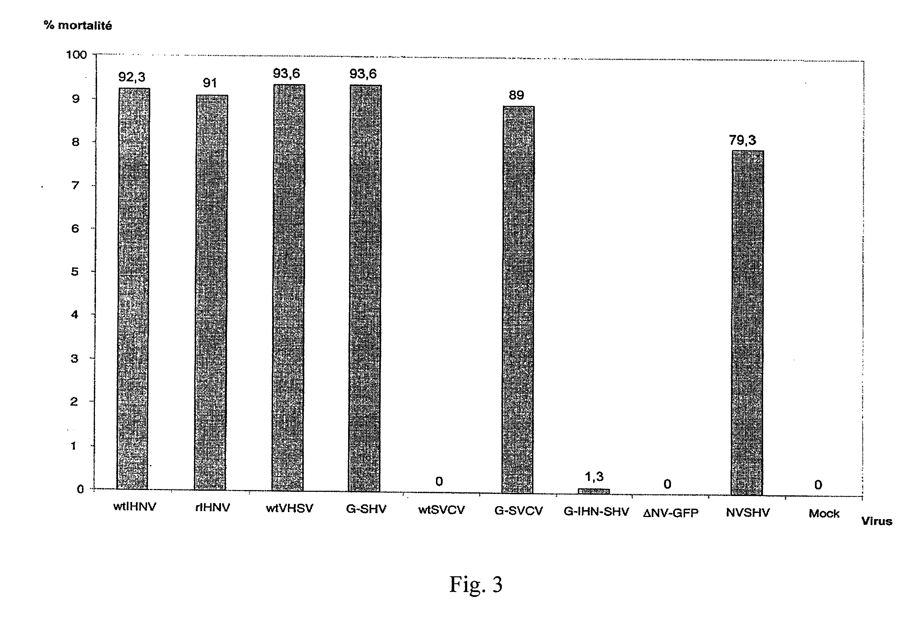

Les virus utilisés sont les suivants :
wtIHNV : virus NHI sauvage, souche française 32/87 ;
wtVHSV : virus SHV sauvage, souche 07-71 ;
wtSVCV : Virus de la virémie printanière de la carpe (vesiculovirus incapable de se multiplier chez la truite), souche Fijan (FIJAN et al., Veterinarski archiv (Zagreb), 41, 125138, 1971 ; séquence complète : HOFFMAN et al., GenBank AJ318079) ;
rIHNV : virus NHI de type sauvage produit comme décrit à l'Exemple 1 ;
G-SHV : virus NHI recombinant portant le gène G du virus SHV à la place du gène G de NHI ;
G-SVCV : virus NHI recombinant portant le gène G du virus SVCV à la place du gène G de NHI ;
G-IHN-SHV : virus NHI recombinant portant le gène G du virus SHV à la place du gène NV de NHI ;
ΔNV-GFP : virus NHI recombinant portant le gène de la GFP à la place du gène NV de NHI ;
NVSHV : virus NHI recombinant portant le gène NV du virus SHV à la place du gène NV de NHI.
Les résultats sont illustrés par la Figure 3.
Légende de la figure 3 : En abscisse : virus utilisé ; Mock: alevins non infectés
En ordonnée : mortalité cumulée à 28 jours post-infection (en pourcentage du nombre initial d'alevins)

Ces résultats montrent que la pathogénicité des virus est liée à la présence du gène NV ; tous les virus contenant ce gène entraînent une mortalité élevée des alevins ; en revanche tous les virus chez lesquels le gène NV a été inactivé ont perdu leur pouvoir pathogène.

### Propriétés vaccinales

Des poissons (alevins de truites) immunisés par balnéation, dans les mêmes conditions que lors des essais de pathogénicité, avec le virus rNHI-ΔNV-GSHV ou le virus rNHI-ΔNV-GFP, ont été soumis 1 mois plus tard à une infection réalisée par balnéation avec les virus sauvages VNHI et VSHV, à raison de 10⁴ à 5 x 10⁴ UFP/ml.

Aucune mortalité significative n'a été observée dans le cas des poissons immunisés avec le virus rNHI-ΔNV-GSHV. Les poissons immunisés avec le virus rNHI-ΔNV-GFP sont également protégés contre le virus VNHI. En revanche, ils meurent entre 7 et 15 jours après l'infection avec le virus VSHV.

Ces résultats montrent que l'immunisation avec le virus rNHI-ΔNV-GSHV protège efficacement à la fois contre le virus sauvage VNHI et contre le virus sauvage VSHV.

## Revendications

1. Virus de la septicémie hémorragique virale (SHV) recombinant dont le gène de la protéine NV est inactivé, pour l'utilisation comme médicament.

2. Virus SHV recombinant dont le gène de la protéine NV est inactivé, **caractérisé en ce qu'**il contient dans son génome au moins un gène hétérologue codant pour une glycoprotéine de capside ou d'enveloppe d'un virus pathogène des poissons.

3. Virus SHV recombinant selon la revendication 2, **caractérisé en ce que** ledit gène hétérologue est inséré à l'emplacement du gène NV.

4. Virus SHV recombinant selon la revendication 3, **caractérisé en ce que** le gène de la protéine NV dudit virus est inactivé, et remplacé par le gène de la glycoprotéine G d'un virus NHI.

5. Molécule d'ADN complémentaire (ADNc) du génome d'un virus SHV recombinant selon une quelconque des revendications 2 à 5.

6. Vecteur recombinant comprenant une molécule d'ADNc selon la revendication 6.

7. Vaccin **caractérisé en ce qu'**il comprend un virus SHV recombinant selon une quelconque des revendications 1 à 5.

8. Vaccin selon la revendication 7 pour la prévention des infections à novirhabdovirus chez les poissons.


SEQUENCE LISTING

<110>  INSTITUT NATIONAL DE LA RECHERCHE AGRONOMIQUE
       BREMONT, Michel
       BIACCHESI, Stéphane
       LEBERRE, Monique
       THOULOUZE, Maria-Isabel

<120>  UTILISATION DE NOVIRHABDOVIRUS MODIFIES POUR L'OBTENTION DE
       VACCINS

<130>  MJPll-F539/114EPdiv

<150>  FR0205702
<151>  2002-05-07

<160>  4

<170>  PatentIn version 3.5

<210>  1
<211>  27
<212>  DNA
<213>  Artificial sequence

<220>
<223>  Description de la séquence artificielle: amorce

<400>  1
cccgggtcag gaggtgagcc cagagcc                                          27


<210>  2
<211>  30
<212>  DNA
<213>  Artificial sequence

<220>
<223>  Description de la séquence artificielle: amorce

<400>  2
actagtatgg cgacccaacc cgggctcagc                                       30


<210>  3
<211>  32
<212>  DNA
<213>  Artificial sequence

<220>
<223>  Description de la séquence artificielle: amorce

<400>  3
cccgggtcag accgtctgac ttctggagaa ct                                    32


<210>  4
<211>  31
<212>  DNA
<213>  Artificial sequence

<220>
<223>  Description de la séquence artificielle: amorce

<400>  4
actagtatgg aatggaacac ttttttcttg g                                     31